Europäisches Patentamt

European Patent Office

Office européen des brevets

(11) Publication number: **0 427 074 A2**

# EUROPEAN PATENT APPLICATION

(21) Application number: 90120652.4

(22) Date of filing: 27.10.90

(51) Int. Cl.5: **C12Q 1/68**, C12P 19/34,
//C07H21/04,C12Q1/70,
G01N33/53

(30) Priority: 09.11.89 US 433947
23.08.90 US 569992

(43) Date of publication of application:
15.05.91 Bulletin 91/20

(84) Designated Contracting States:
AT BE CH DE DK ES FR GB GR IT LI LU NL SE

(71) Applicant: MOLECULAR DIAGNOSTICS, INC.
400 Morgan Lane
West Haven, CT 06516(US)

(72) Inventor: Dattagupta, Nanibhushan
470 Prospect Street
New Haven, CT 06511(US)

(74) Representative: Dänner, Klaus, Dr. et al
c/o Bayer AG Konzernverwaltung RP Patente
Konzern
W-5090 Leverkusen 1 Bayerwerk(DE)

(54) Nucleic acid amplification employing transcribable hairpin probe.

(57) Specific nucleic acid sequences are amplified through the use of transcribable hairpin probes. The probe comprises a single stranded self-complementary sequence which, under hybridizing conditions, forms a hairpin structure having a functional promoter region, and further comprises a transcribable sequence extending from the 5′ end of the hairpin sequence and a probe sequence which may be comprised in the transcribable 5′ sequence or in a sequence extending from the 3′ end of the hairpin sequence. The hairpin form of the probe is transcribable in the presence of a suitable RNA polymerase and appropriate ribonucleoside triphosphates (rNTPs). Amplification is accomplished by hybridizing the desired target nucleic acid sequence with the transcribable probe, transcribing substantially only probe which has become hybridized to the sequence of interest, and allowing transcription to proceed until a desired amount of RNA transcription product has accumulated. Optional second stage amplification of the RNA transcripts can produce higher levels of amplification. The amplification method is particularly useful in assays for the detection of particular nucleic acid sequences.

EP 0 427 074 A2

# NUCLEIC ACID AMPLIFICATION EMPLOYING TRANSCRIBABLE HAIRPIN PROBE

## BACKGROUND OF THE INVENTION

The present invention relates to methods for amplifying nucleic acid sequences. In particular, the invention concerns methods for detecting the presence of a particular nucleic acid sequence with high sensitivity.

The detection of specific nucleic acid sequences is gaining rapid importance in a variety of fields, particularly in the field of medical diagnosis. Nucleic acid hybridization methods provide assays for detecting nucleic acid sequences of medical significance, such as DNA or RNA sequences indicative of genetic diseases, cancer, and bacterial and viral infections. Nucleic acid hybridisation assays are based on the very specific base pairing that is found in hybrids of DNA and RNA. Base sequences of analytical interest appearing along a strand of nucleic acid can be detected very specifically and sensitively by observing the formation of hybrids in the presence of a probe nucleic acid known to comprise a base sequence that is complementary with the sequence of interest.

It is evident that for hybridization assays to attain their full analytical potential, methods for increasing the sensitivity of detection even further are needed. Considerable efforts have been applied to this aspect in recent years and a number of different approaches have been conceived and developed. Particularly promising are approaches based on the biochemical amplification of the target nucleic acid sequence or its complementary signal sequence. While detection systems each have their own sensitivity limits, biochemical systems have been developed which can make millions and millions of copies of the target or signal sequences thereby to extend the effective sensitivity limits of such detection systems by many orders of magnitude.

One such nucleic acid amplification method is that known as the polymerase chain reaction method, or PCR, which is described in U.S. Pat. Nos. 4,683,195 and 4,683,202. PCR employs a pair of specific oligonucleotide as primers for the two complementary strands of the double stranded form of a target sequence. The primers are chosen such that they form specific hybrids at the opposite 3′ ends of the complementary target strands. Using a thermostable DNA polymerase, the primers are extended synthetically in correspondence with the target sequences. A thermal cycling process is required in order to form specific hybrids and, after extension, to denature the hybridized, extended strands for further primer hybridization and extension. Repeating the process several times results in a geometric amplification of the amount sof the target sequences in the mixture.

A variation of PCR is the ligase chain reaction (LCR) described in European Patent Publication 320,308. This method requires at least four separate oligoprobes, two of which hybridize to opposite ends of the same target strand such that when they are hybridized to the target sequence their respective 3′ and 5′ ends are juxtaposed for ligation. The third and fourth probes hybridize with the first and second probes to form, upon ligation, fused probes which can be denatured and detected.

Another known amplification method is described in PCT Publication No. 88-10315 and will be referred to as the transcription amplification system or TAS. Similar methods are described in European Patent Publication No. 310,229. As in PCR, TAS uses pairs of oligoprimers to hybridize with opposite ends of a desired target sequence. The primers are chosen such that the extension products, after either a single extension or multiple cycles as in PCR, comprise transcription promoter sites. In the presence of a suitable promoter specific polymerase and ribonucleoside triphosphates (rNTPS, the extension products are themselves further amplified by transcription.

The Qβ replicase (QβR) method described in PCT Publication No. 87-06270 uses a specific RNA probe which is capable of specific transcription by a replicase enzyme. The method has linear reaction kinetics and requires the design and synthesis of RNA probes with replicase initiation sites.

While all of these methods yield amplification of a target nucleic acid sequence, none are without complexities which are undesirable for the general and unsophisticated user. Many of the prior art methods require multiple incompatible steps that can be accomplished only by cumbersome manual procedures or complex and expensive instruments for automating the many manipulations required. Further, many require the preparation of multiple sophisticated reagents which limits the ready application of the methods to different target sequences.

Unrelated to the above pursuits, there have been studies of a variety of synthetic and naturally occurring DNA and RNA structures and their functions. One such structure is that known as the hairpin in which self-complementary regions in a single polynucleotide hybridize under suitable conditions to form looped structures whose shape resembles a common hairpin. Such hairpin structures are known to occur naturally in many organisms, particularly in RNA secondary structures, however, their functional role is at this point not well established. The physical chemistry of hairpin structures has been described - Cantor and Schimmel, Biophysical Chemistry, Part III, p. 1183, M.R. Freeman & Co. (San Francisco 1980).

The literature on this subject is incomplete and contradictory. For example, there are predictions that hairpins may provide a transcription termination signal - Jendrossek et al, J. Bacteriol. 170:5248 (1988) and Walker et al, Biochem. J. 224:799 (1984). Hairpin structures resembling known rho dependent transcription termination signals have been observed following the unc operon and glms of E. coli. On the other hand, pallindromic sequences capable of forming stable hairpin forms have been found around the transcription initiation site of beta amyloid precursor gene - La Fauci et al, Biochem. Biophys. Res. Commun. 159:297 (1989).

The use of hairpin structures in the synthesis of DNA from oligonucleotides and in the labeling of oligonucleotides is proposed in European Patent Publication 292,802 and by Sriprakash and Hartas, Gene Anal. Techn. 6:29-32 (1989). In addition, Krupp and Söll, in FEBS Letters 212:271 (1987) and in "Nucleic Acid Probes", ed. Symons (CRC Press, Bacon Raton, FL, 1989) pp. 21 & 22, describe the use of a hairpin structure to make labeled RNA transcripts from an M13 vector/T7 RNA polymerase system.

## SUMMARY OF THE INVENTION

The present invention provides a method and means for amplifying a particular nucleic acid sequence of interest (target sequence) using a probe which is inherently transcribable, and accordingly, capable of providing multiple copies of RNA corresponding in base sequence to (i) a portion or the entirety of the target sequence or iii) a preselected sequence distinct from the target sequence. The transcribable probe has three principal parts, (1) a single stranded self-complementary sequence capable of forming, under suitable hybridizing conditions, a hairpin structure having a functional promoter region, and (2) a transcribable sequence extending from, and forming part of the same nucleic acid molecule with, the 5' end of the self-complementary sequence, and (3) a single stranded probe sequence substantially complementary to the sequence of interest. The probe sequence can be comprised in the 5' transcribable sequence or can be part of a sequence extending from the 3' end of the hairpin sequence, in which latter case, it is not substantially complementary to the 5' transcribable sequence.

Under suitable hybridizing conditions, the self-complementary region of the probe forms a looped, self-hybridized structure commonly referred to as a hairpin loop, or simply, hairpin. The base sequence of the self-complementary region is selected such that upon formation of the hairpin, a desired double stranded promoter sequence is formed operably linked to the transcribable region. Thus, the hairpin form of the probe as such is transcribable in the presence of a suitable RNA polymerase and the required ribonucleoside triphosphates (rNTPs). Where the probe sequence is comprised in the 5' transcribable sequence, the sequence of bases in the RNA transcription product will accordingly be complementary with the probe sequence. Since, in such case, the probe sequence is complementary with the target sequence, transcription of the hairpin probe produces multiple RNA transcripts having the same base sequence as the target nucleic acid. Where the probe sequence is comprised in a sequence extending from the 3' end of the hairpin region, the transcribable sequence can be selected to provide an RNA transcript having any useful sequence, for example, a sequence that is common for a series of probes for detecting a series of target sequences in order to enable use of a common, or universal, detection probe and system.

After hybridizing the target sequence with the present probe in a liquid mixture, the next step in the method is the transcription of probe which has become hybridized with the target without substantial transcription of any probe which is not hybridized to the select target sequence (unhybridized probe). Since the probe is transcribable irrespective of whether it is hybridized or unhybridized, it is necessary, in order to amplify and/or detect the target sequence specifically, to distinguish hybridized probe from unhybridized probe. Any suitable scheme may be applied for this purpose.

For example, one approach is based on separation of hybridized from unhybridized probe and then adding the polymerase and rNTPs only to the separated hybridized probe fraction. Another approach involves the use of a second probe which is complementary to a second sequence in the nucleic acid to be amplified. The base sequence of the second probe is selected such that its 3' end is within ligating distance of the 5' end of the transcribable probe when both such probes are hybridized with the target nucleic acid. After hybridization, the 3' end of hybridized second probe is suitably ligated to the 5' end of hybridized transcribable probe. Thus, transcription of the combined sequence encompassed by the now joined

3

transcribable and second probes occurs only if the target sequence is present and becomes hybridized with the such probes.

Transcription can be allowed to proceed for any desired length of time with the accumulated transcription product producing an amplification of the target sequence. Where the target sequence is of analytical interest, high sensitivity detection of the target sequence can be accomplished by amplifying the target in the manner of the present invention and then suitably detecting the accumulated transcription product. Any number of conventional approaches can be taken in order to detect the accumulated RNA transcription product.

For example, the rNTPs added for transcription can comprise a detectable label and, after separating resulting labeled transcription product from the unused labeled rNTPs, the label is detected in the separated product fraction. Another approach is to detect the transcription product by hybridization with a detectable nucleic acid probe and detecting the resulting hybrids in any conventional manner, e.g., using labeled probe or anti-hybrid selective antibody such as anti-DNA/RNA.

Amplification can be further increased by applying a secondary or second stage amplification of the generated RNA transcription product. A variety of methods are appropriate for this purpose, representative examples of which are described in more detail infra.

The present amplification method provides a number of significant advantages over the methods of the prior art. First, the present method requires, in its most general form, but a single probe component as opposed to the need for multiple probes, such as oligoprimers, in many of the prior art methods such as PCR, TAS, and LCR described above. Further, the present probe can be presented to the target sequence in an intact and stable transcribable form, that is, it is not required to synthesize the transcribable form of the probe in situ such as in TAS. Moreover, there is no need for time consuming and and cumbersome thermal cycling as with PCR and LCR. Unlike the QβR method described above, the present probe is a simple extended single stranded nucleic acid rather without complex tertiary structure. Other advantages will be evident to the worker in the field.

## BRIEF DESCRIPTION OF THE DRAWINGS

Figs. 1-3 are illustrations of several different forms of the transcribable probe of the present invention, including the hairpin form and the dimer form thereof.

Fig. 4 is a diagram illustrating the use of a transcribable probe of the present invention in amplifying a target nucleic acid sequence with a separation step being used to differentiate hybridized from unhybridized probe.

Fig. 5 is a diagram illustrating the use of the present probe in amplification without the need for physical separation of hybridized and unhybridized probe. This illustrated method uses a second probe with ligation of the transcribable and second probes when hybridized with the target sequence.

Fig. 6 is a diagram illustrating the use of a probe of the present invention wherein the probe sequence is comprised in a sequence extending from the 3' end of the hairpin sequence. In the particular method illustrated, hybridized probe is distinguished from unhybridized probe by using an immobilized form of the probe and a restriction digest specific for hybridized probe.

Fig. 7 shows an autoradiogram of a polyacrylamide gel demonstrating transcription of hairpin oligonucleotides.

Figs. 8 and 9 are bar graphs depicting the results of amplification experiments described in the Examples below.

## DESCRIPTION OF THE PREFERRED EMBODIMENTS

### The Transcribable Probe and Its Preparation

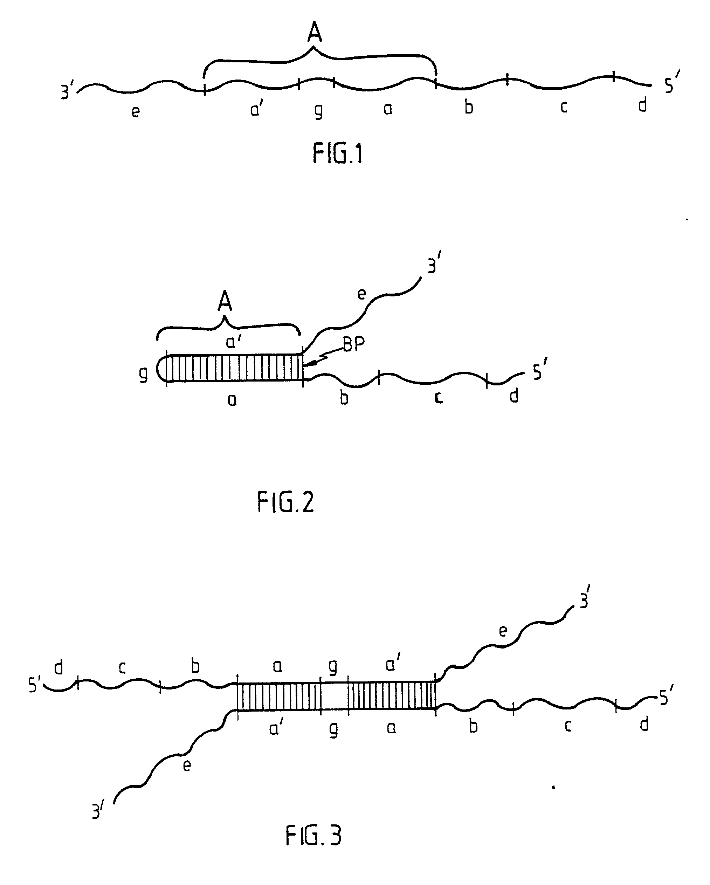

The transcribable probe of the present invention comprises at least three principal parts joined together in a single polynucleotide. With reference to Figs. 1-3 of the drawings, the first part is sequence of bases A that comprises self-complementary portions a and a' separated by a loop sequence g. Under hybridizing conditions, sequences a and a' self-anneal to form a looped, hairpin structure illustrated in Fig. 2 with the lines BP representing the base pair bonds formed between the self-complementary sequences. Sequences a and a' are selected in order that the resulting looped region A thereby comprises a functional promoter or transcription initiation site. The second principal part of the probe of the present invention is transcribable sequence c which is operatively linked directly or through an intervening sequence b to the 5' end of promoter region A. The third principal part of the probe is a probe sequence that is selected to be hybridizable with a target sequence to be amplified or detected and can be comprised in transcribable sequence c or as part of sequence e extending from the 3' end of the hairpin region. The probe can, in general, additionally comprise 5' and/or 3' flanking sequences such as are illustrated in Figs. 1 and 2 as sequences d and e.

The hairpin form of the probe as depicted in Fig. 2 is transcribable in the presence of a cognate polymerase and the rNTPs required for 3'-5' transcription of transcribable sequence c and any intervening sequence b. Depending upon the concentration of probe present and reaction conditions, a functionally transcribable dimeric form can also be formed (Fig. 3). Self-complementary region A can potentially hybridize not only with itself, but also with a second probe molecule to form a dimer through hybridization of complementary regions a and a' on separate probe strands. Such dimer, like the hairpin form depicted in Fig. 2, comprises a functional promoter in region A and, in the presence of cognate polymerase and rNTPs, will produce 3'-5' transcription of transcribable sequence c and any intervening and/or flanking sequences b and d. Accordingly, when reference is made herein to the "hairpin form" of the present probe, it will be understood that the functionally equivalent dimeric form is intended as well.

The promoter formed by hybridization of hairpin region A of the probe can be any double stranded nucleic acid (e.g., DNA or RNA) sequence that corresponds with, and is recognized by, a polymerase enzyme which, as is known in the art, binds to the promoter and thereby initiates 3'-5' transcription. The length of the self-complementary sequences a and a' which form the promoter, while not critical to the operation of the present invention, will generally fall between about 7 and about 200 bases, and more commonly between about 10 and about 50 bases. In general, any promoter for which a suitable polymerase is known and available can be used in the present invention. Usually, the probe will be composed of DNA which is transcribable with DNA-dependent RNA polymerases, that is, polymerases that act upon a DNA template to produce RNA transcripts. However, RNA probes transcribable with RNA-dependent RNA polymerases (such as produced in certain viruses, e.g., retrovirus and picornovirus) can be used. Useful promoters include, among others, those recognized by RNA polymerases produced in bacteriophages, such as T7, T3, and SP6 phage.

Exemplary base sequences having promoter function with respect to such phage polymerases are ($n$ = 2 to 50; the sequences are shown in their self-hybridized, hairpin form and do not necessarily represent the minimum sequences required for promoter function):

**For T7**   ⌐TAATACGACTCACTATAG-3'
$T_n$
⌐ATTATGCTGAGTGATATC-5'

**For T3**   ⌐ATTAACCCTCACTAAAGGGA-3'
$T_n$
⌐TAATTGGGAGTTAGCTTTCC-5'

**For SP6**   ⌐CATACGATTTAGGTGACACTATAG-3'
$T_n$
⌐GTATGCTAAATCCACTGTCATATC-5'

The probe sequence, whether comprised in sequence c or e, can be any sequence which is capable of hybridizing with the target sequence of interest. As is known in the art, the degree of homology between the probe and target sequences will depend upon the specific needs of the user. Where a high degree of specificity is needed or desired, perfect or near perfect homology may be required, such in the case of the detection of single base pair mismatches. However, in the normal case, a degree of nonhomology can be tolerated without sacrificing required specificity, amplification, or detection of the target sequence. Thus, the term "complementary" or "hybridizable" will be used herein to describe the desired or necessary degree of homology between the probe sequence and the target. The length of the probe sequence is, in general, not critical. However, in order to obtain rapid and strong hybridization with a selected target sequence, normally probe sequences of at least about 10 bases will be used.

The loop sequence g in the probe which links the self-complementary, hairpin forming sequences a and a′ can be of any composition and length so long as it does not substantially impede or inhibit the formation of the transcribable hairpin or dimer structures. Normally, loop sequence g will be selected to be substantially noncomplementary with itself, e.g., will be composed of a heteropolymeric or homopolymeric DNA or RNA, such as a chain comprising poly T, poly A, poly C, or poly G, or combinations, and will be at least about 2 bases long to allow suf ficient steric freedom for loop formation. More usually, loop sequence g will be between about 4 and about 50 bases in length.

The transcribable sequence is operably linked, as is known in the art, to the 5′ end of the promoter in order that upon initiation of transcription, the sequences extending from the 5′ end of the promoter are functionally transcribed by the polymerase to form RNA transcripts which comprise a sequence corresponding to the transcribable sequence. As indicated above, the transcribable sequence c can be separated by an intervening sequence b providing that such intervening sequence retains operable linkage with the transcribable sequence, e.g., it does not contain a transcription termination site, is not so long as to significantly reduce the efficiency of transcription of the transcribable sequence, and does not introduce significant nonspecific hybridization. In certain situations it will be desirable to include such an intervening sequence for stability or detection purposes. It has also been found that the efficiency of transcription can be dependent upon the sequence presented in the first few (e.g., 3 to 5) nucleotides in the transcribable sequence. In particular, it has been found that highly efficient transcription using T7 RNA polymerase can be attained where the initial sequence is CCCTC.

Likewise, the probe can comprises flanking sequences at one or both of its 3′ and 5′ ends. Sequences d flanking the 5′ terminus will be transcribed to an extent dependent upon the efficiency of the RNA polymerase used and the conditions of transcription. In certain situations such flanking sequence can be used to advantage in separation or detection.

The transcribable probe of the present invention can be prepared by any suitable method. Such methods, in general, will include oligonucleotide synthesis and cloning in a replicable vector. Methods for nucleic acid synthesis are well kown in the art. For example, in "Oligonucleotide Synthesis: A Practical Approach", ed. M.J. Gait, IRL Press (Oxford 1984) there are described several different methods of oligonucleotide synthesis and purification and analysis of resulting products. In an automated synthesizer, one starts with a 5′-protected, immobilized (through the 3′-hydroxyl) nucleoside residue. After 5′-deprotection, a phosphodiester linkage is introduced by reaction with a 3′-phosphoramidite, 5′-protected nucleoside residue. The phosphite linkage is then oxidized to a stable linkage and the process is cycled with desired nucleoside residues for the sequence to be synthesized. In place of this phosphite triester phosphotriester method, one can also use a solid phase approach. Also, the synthesized nucleic acid can be further modified by synthetic methods (e.g., as described in U.S. Pat. No. 4,818,681). Cloning of nucleic acids in an amplifying vector is also well known in the art (see Maniatis et al, Molecular Cloning, Cold Spring Harbor (1982). When cloned in a double-stranded vector, strand separation may be necessary in order to use the product as a probe.

Target Amplification Methods

The first step in amplifying a particular target nucleic acid according to the present method is the hybridization of such target with the transcribable probe in a suitable liquid mixture. Such hybridization will be performed under suitable conditions well known in the art.

The sample suspected or known to contain the intended target nucleic acid may be obtained from a variety of sources. It can be a biological specimen, a food or agricultural sample, an environmental sample, and so forth. In applying the present method to the detection of a particular nucleic acid sequence in the assistance of medical diagnosis, the test sample can be a body fluid or exudate such as urine, blood, milk, cerebrospinal fluid, sputum, saliva, stool, lung aspirates, throat or genital swabs, and the like. As discussed elsewhere in more detail herein, the target nucleic acid can be RNA or DNA. In some circumstances, it will

6

be necessary or desirable to treat the test sample to release and/or extract the target nucleic acid for hybridization, and/or, when the target nucleic acid is presented in double stranded form, to denature and render such in hybridizable single stranded form by means well known in the art. Although not generally necessary, it can be desirable, such as to increase the overall efficiency of hybridization, to subject the target nucleic acid to restriction digest.

Since the present probe is designed in such a manner that it is capable of undergoing transcription whenever it is in the hairpin form, whether or not hybridized to the target, it is necessary to take steps to limit transcription or detection substantially only to probe that hybridizes with the target sequence. Any number of formats can be devised for the purpose of transcribing probe that has become hybridized with the target without transcribing substantially any of the hybridized probe. Approaches that are particularly advantageous are methods based on separation of hybridized probe from unhybridized probe, methods based on the use of a second probe which is ligatable to the transcribed 5′ end of the probe only if hybridized with the target, and methods based on use of a restriction enzyme which digests only hybridized probe to release a transcribable product.

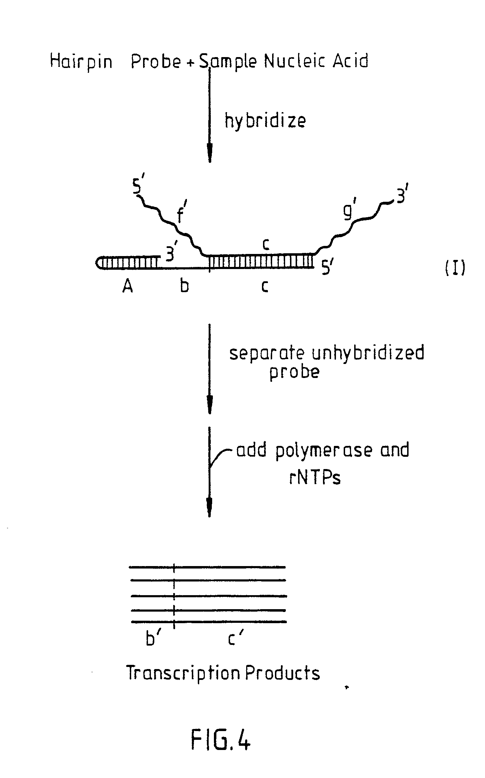

Formats which are based on the separation of hybridized from unhybridized probe are generally illustrated by the Fig. 4 diagram. Hybridization of the present hairpin probe with the target sequence present in a sample of nucleic acids produces hybrids (I) in which a probe sequence c in the probe is hybridized with its complementary target sequence $\bar{c}′$. In Fig. 4, the target nucleic acid is shown with flanking regions f′ and g′ in addition to the target sequence $\bar{c}′$ and, the transcribable probe is depicted without regions flanking the hairpin region A or the probe sequence c, although, as discussed herein, other variations are also possible. Further, although not shown, these formats are also applicable where the probe sequence is comprised in a sequence extending from the 3′ end of the hairpin loop. After separation of unhybridized probe and addition of the polymerase and rNTPs, transcription proceeds with the generation of multiple transcripts having the combined sequence $\bar{b}′\bar{c}$ (where b′ is complementary to b).

Numerous formats are possible for separating hybridized from unhybridized probe. Many such techniques are well known in the literature and others will be devised in the future. Any such technique can be applied to the present invention. After separation, the hybridized probe fraction will be contacted with the appropriate polymerase and the rNTPs required for transcription. Following are descriptions of just a few of the possible separation schemes that can be used in the present invention for this purpose.

(1) Immobilization of target - In this approach, the target sequence is immobilized either before or after hybridization with the transcribable probe and resulting immobilized hybrids are readily separated from unhybridized probe. As is well known in the art, target nucleic acid can be immobilized prior to hybridization by such techniques as noncovalent or covalent attachment to a suitable solid phase, e.g., nitrocellulose or activated nylon membranes or reactive or adsorbent latex particles. Immobilization can also be accomplished by hybridization with a separation probe, that is, a probe which is immobilized or is immobilizable and comprises a sequence hybridizable with a sequence in the target nucleic acid which is nonoverlapping with the sequence that is hybridizable with the transcribable probe. In use, dual hybridization occurs with the target nucleic acid becoming hybridized to both the separation probe and the transcribable probe. The resulting immobilized hybrids are readily separable from unhybridized transcribable probe.

(2) Modifying the target for post-hybridization immobilization - Rather than immobilizing the target sequence directly, it is known to modify the target prior to hybridization in such a manner that the target can subsequently serve as a site for immobilization. This also permits hybridization to take place under favorable solution kinetics. The target can be modified to be immobilizable in a number of known ways. Normally, a reactive site is introduced into the target which is capable of forming a stable covalent or noncovalent bond with a reaction partner. Immobilization can then be accomplished at the desired time by adding an immobilized form of the reaction partner to bind with the modified target strand. Typical reaction partners are ligands such as biotin and haptens which are capable of specific binding with avidin and anti-hapten antibodies, respectively.

Modification of the target stand in a sample can be accomplished in several different ways as well. One particularly useful approach involves the introduction of a suitable reactive site into the target strand by reaction with a reactive site-functionalized nucleic acid binding agent such as photoreactive intercalating agents (for example, see U.S. Pat. No. 4,737,454). Particular examples of such modifying agents are bifunctional conjugates comprising a photochemically reactive form of a psoralen coupled to a ligand such as biotin or a hapten.

(3) Use of anti-hybrid antibody reagents -Another known method for separating hybrids from unhybridized probe involves the use of antibody reagents which are prepared or selected to be selective for binding hybrids over single stranded nucleic acids. A variety of such anti-hybrid reagents are known in the literature, including anti-DNA/RNA and anti-RNA/RNA antibodies (U.S. Pat. No. 4,833,084 and European

7

Patent Publication No. 163,220), anti-DNA/DNA antibodies (U.S. Pat. No. 4,623,627), and antibodies to intercalated duplexes (U.S. Pat. No. 4,563,417). Antibodies specific for PNA/DNA hybrids are particularly useful since the transcribable probe will commonly be composed of DNA and the target nucleic acid of RNA. The antibody reagent can be in the form of whole antibodies, antibody fragments, or aggregated forms thereof, provided that it comprise an active antibody combining site. Further, the antibody reagent can be added in an immobilized form or in a form that is immobilizable, i.e., which upon further reaction can be rendered immobilized. These variations are all well known in the art.

(4) Dual hybridization - One can also use a second immobilized or immobilizable (separation) probe in addition to the hairpin probe for the purposes of separating hybridized hairpin probe from unhybridized hairpin probe (see European Patent Publication Nos. 130,515 and 192,168). The probe sequence in the hairpin probe and the probe sequence in the separation probe are selected to be hybridizable with mutually exclusive portions of the target sequence. Thus, upon hybridization, the target sequence serves as a bridge between the hairpin probe and the separation probe. The separation probe can be bound or attached to a suitable solid support or can comprise a reactive site for a reaction partner, preferably a binding site (e.g., a biotin or a hapten residue) for a binding partner (e.g., avidin or anti-hapten antibody, respectively). After hybridization, the resulting solution is contacted with an immobilized form of the reaction partner whereby dual hybrids become immobilized.

Methods can also be used which do not rely upon physical separation of hybridized and unhybridized probe. Such methods will rely upon the strategy of designing a transcription system that produces a distinct RNA transcript when the template for the polymerase is or has been hybridized with the target sequence compared to the RNA transcript produced by the unhybridized probe.

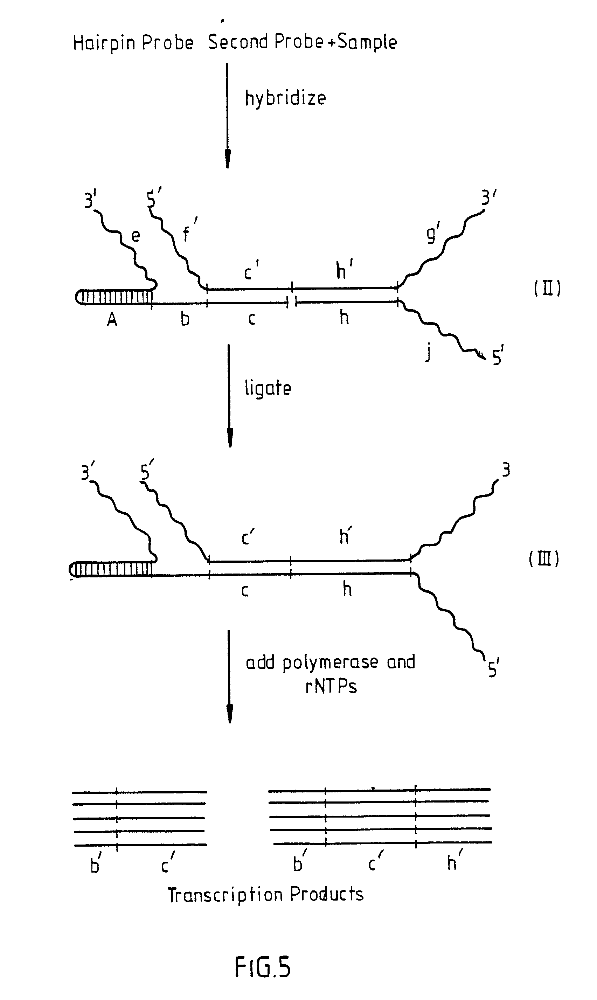

One such approach involves the use of a second probe and is illustrated in Fig.5. The second probe is designed or selected to comprise a sequence $h$ that is complementary to a second sequence $h'$ in the target nucleic acid. Sequence $h$ is such that the $3'$ terminus of the second probe becomes localized within ligating distance of the $5'$ terminus of the transcribable probe (at the $5'$ terminus of probe sequence $c$) in hybrids (II) formed between the target sequence on the one hand and and the two probes on the other. Sequence $h$ is also depicted as comprising flanking region $j$, however, it will be appreciated that such flanking region is optional. Further, in this illustration, the transcribable probe, while not comprising a region flanking probe sequence $c$ (in order to permit ligation to sequence $h$), is shown with flanking region $e$ extending from the $3'$ end of the hairpin region A. After hybridization, the $3'$ end of the second probe and $5'$ end of the transcribable probe are ligated to one another to form transcribable ligation products (III). Subsequent transcription of the transcribable ligated nucleic acid produces an RNA product which is distinct from the transcript produced by the unligated transcribable probe, the former transcript having the sequence $b'c'h'$ while the latter transcript would have the shorter sequence $b'c'$. Ligation can be accomplished in any conventional manner (see Maniatis et al, supra).

While, as stated previously, that physical separation of hybridized and unhybridized probe is in principle not necessary with this approach, such separation can be advantageous to further reduce background RNA. Any number of ways to accomplish such, separation will be evident to the practitioner. For example, one can modify, e.g., with a binding site such as biotin, the sample sequence, the second probe, or the transcription product such that same can be isolated by addition of a suitable, e.g., immobilized, binding partner. Biotinylation of the sample sequence provides the additional advantage of inhibiting, nonspeci ic transcription of such sample sequence.

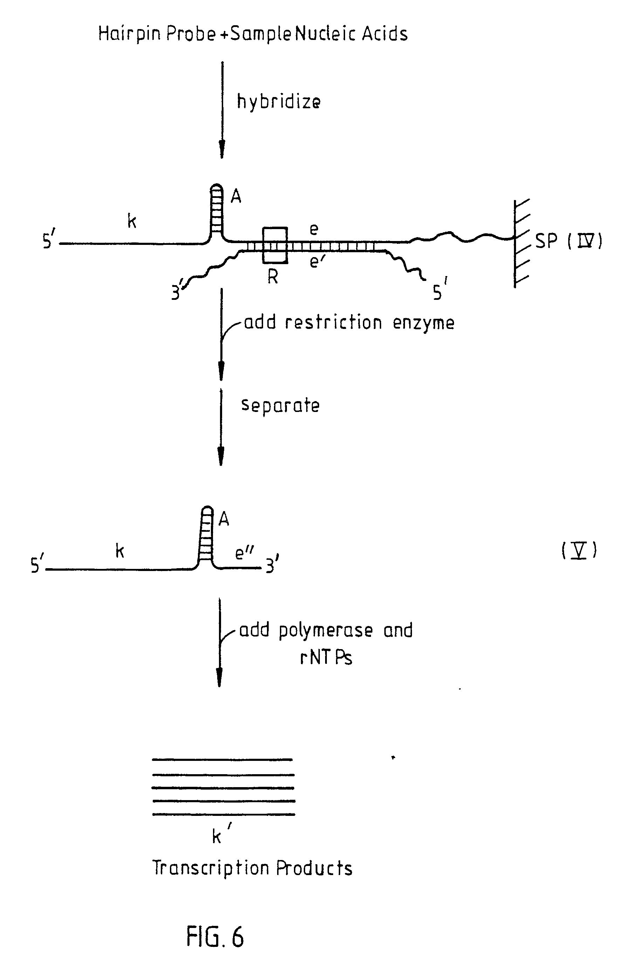

A third method of accomplishing transcription of substantially only hybridized probe is based on use of a restriction enzyme which digests only hybridized probe to release a transcribable product. Such a method is illustrated in Fig. 6 using a transcribable hairpin probe wherein a probe sequence $e$ is positioned on the $3'$ end of the hairpin region A. The probe further comprises transcribable sequence $k$ operably linked to the 59179 end of the hairpin promoter. In the particular method illustrated, the transcribable probe is immobilized by being linked at its $3'$ end to a solid phase SP. The probe region $e$ is selected such that upon hybridization with complementary sequence $e'$ in the sample nucleic acids, a restriction site R is formed in hybrid products (IV). Addition of a restriction enzyme specific for site R will result in cleavage and release of products (V) which can be readily separated from hybridized solid phase probe. Released products (V) comprise a short $3'$ sequence $e''$ produced by the action of the restriction enzyme and retain transcribable sequence $k$. Upon addition of a suitable polymerase and the required rNTPs, transcription products having corresponding sequence $k'$ are produced.

The ability to create a specific restriction site upon hybridization can be applied to other variations of the present method. For example, a probe can be designed to have a $5'$ transcribable sequence which includes the probe sequence and which is linked to a solid phase for immobilization purposes. Upon hybridization and digestion, the cleaved probe product will comprise a shortened, transcribable $5'$ sequence. Thus, resulting transcript products will be uniquely identifiable to the hybridization event. A further modification involves the inclusion of a 3′ sequence that is complementary with a non-probe 5′ sequence on the hairpin probe or with a separate oligonucleotide such that, upon hybridization, multimers of the hairpin probe are formed by linkage of individual hairpin probes by the complementary sequences in their 3′, 5′, and/or the oligo sequence. Release of such multimer transcribable product occurs only when hybridization with the target sequence creates the required restriction site in the hybridization products.

Transcription is initiated by addition of polymerase and the required rNTPs to the liquid mixture that contains the hybrids comprising the transcribable probe. Under suitable conditions, the synthesis of RNA transcripts will proceed in a continuous manner providing that sufficient amounts of rNTPs are present. Normally, a ribonuclease inhibitor will be included in the transcription reaction mixture in order to avoid undesirable degradation of RNA transcripts by any ribonuclease contamination. Transcription is allowed to proceed for a predetermined period of time or until a detectable or desirable amount of RNA transcript has accumulated. The amount of RNA transcript produced in a given period of time will be proportional to the amount of target sequence present in the original sample. The accumulated transcription product thus serves as an amplification of the target sequence. Transcription can then be terminated by any conventional means such as inactivation of the polymerase or removal of reactants from the mixture.

Further amplification of the RNA transcription products can be accomplished in a number of ways, for example, by the use of replicases such as Qβ replicase or replicase from brome mosaic virus. Also, a separate set of hairpin probe/second probe pairs can be used where the hairpin probe (distinct from the original hairpin probe) and the second probe hybridize with adjacent sequences in the RNA transcription product. After hybridizing the hairpin probe/second probe pairs to transcripts, the hybridized pair is ligated to form transcribable nucleic acids which themselves will produce additional RNA transcript in the presence of the polymerase. Further, the RNA transcripts can be produced to contain a site for immobilization (e.g., by use of ligand, e.g., biotin or hapten, modified rNTPs and immobilization of resulting transcripts by addition of an immobilized form of a ligand binding partner, e.g., avidin or an anti-hapten antibody, respectively), and after being separated from the mixture can be hybridized to a further probe to introduce a promoter site for further transcription. After a few cycles, more than a million-fold amplification is possible.

The following methods in particular are useful for providing a second stage amplification:

(1) Displacing probe from hybrid by RNA - The promoter probe is hybridized with its complementary DNA which is immobilized onto a solid support or hybridized to an inmobilizable support. The immobilized or immobilizable support is brought into contact with the product RNA under conditions of specific hybridization. This releases the transcribable probe since RNA will hybridize instead of the DNA because of the stability difference between the RNA-DNA hybrid and DNA-DNA hybrid. After the first stage of transcription, the RNA polymerase activity is destroyed by heating before the mixture is reacted with the immobilized DNA hybrid support under conditions of strand displacement. For every molecule of RNA, one molecule of promoter probe will be produced under the most ideal conditions. By cycling the system it is possible to use RNA to produce more and more transcribable probes and hence secondary amplification of the system. For more effective strand displacement, branch migration can be utilized. In this manner, the displacing DNA molecule has on its 3′ or 5′ end an unhybridized single stranded region where a corresponding part of the RNA initiates RNA-DNA hybrid formation.

It has been assumed that a single strand branch migration phenomenon is involved in transcription of a double stranded DNA. A newly synthesized strand of RNA replaces a DNA strand of the same sequence. The migration rate of a single stranded branch is estimated to be faster than 1,000 base pairs per second. A double stranded branch migration has also been described. This process is about 6,000 base pairs per second at 37° C (Biophysical Chemistry, Cantor & Schimmel, Freeman publication, San Francisco, 1980, vol. III, pp. 1238-1239). From this information it can be easily estimated that a 20 base branch migration displacement should be a very rapid process without any high temperature treatment.

(2) Displacing one strand of the probe - This method is similar to the previous method except that the released probe is not the complete probe, but rather one strand of the probe with one strand of the promoter. Only addition of the other strand makes it transcribable.

(3) Displacing ligatable linker - This method is also similar to the previous methods, however, the released fragment acts as a linker for the ligation of two portions of the promoter probes.

(4) RNA mediated ligation - This method involves the use of RNA product to form a bridge over which ligation of two DNA fragments can occur and is characterized by the advantage of being capable of being performed in solution.

(5) Capture recycling - This method uses ligand-modified (e.g. biotinylated) RNA product of the first transcription as the capturing agent for the promoter probes. At the time of the initial transcription, biotinylated UTP is mixed with the other nucleoside triphosphates for the initial transcription. The biotinylated RNA is then either captured before or after mixing with the complementary promoter probe. When the biotinylated RNA is captured, the first interaction with the complementary DNA promoter is carried out on the solid support. The biotinylated RNA-DNA hybrid is then used for transcription to produce more RNA. If the transcription becomes inefficient from such a hybrid, heat or alkali treatment to release DNA from the hybrid can be carried out before transcription. A similar method can also be performed by using anti-PNA/DNA hybrid antibody capture reaction and dispensing with the step of biotinylating RNA.

(6) RNA mediated copying - This method creates a promoter site by using the transcript as a primer. The primer extension product acts as the transcribable signal. A sequence complementary to the RNA product is cloned into a single stranded phage vector e.g., M13. The transcript is allowed to react with such M13 DNA and the hybrid is then extended using a DNA polymerase and deoxynucleoside triphosphates some of which are labeled for the identification of the product. This process can also produce transcribable sequences for further amplification.

An identical procedure can be followed via a synthetic oligonucleotide instead of a cloned DNA as the template for extension of the transcription product RNA primers.

Promoter-containing DNA is hybridized with the product RNA, extended by using a DNA polymerase and then extended product is transcribed. Starting single stranded RNA does not transcribe. The final product is analyzed by capture with a specific immobilized probe. This process can be adjusted to make as much amplification as needed for a sensitive analysis.

(7) Displaced DNA mediated copying - This method is the same as the previous method except that it uses displaced DNA as the primer for the extension to produce transcribable products.

(8) Immobilized oligo mediated capture - In this method, an oligonucleotide (e.g., half the size of the intact transcript) is immobilized onto a solid support, the product RNA is captured by hybridization and the residual unhybridized portion of the RNA is then used to capture a transcribable hairpin probe.

(9) Use of RNA dependent RNA polymerases - In this method, the initial probe is modified to carry a sequence of single or double-stranded DNA which after transcription produces RNA specific for further amplification by an RNA dependent RNA polymerase (e.g., $Q\beta$ replicase). This sequence is on the 5' end of the probe. The product RNA is used for further amplification without secondary processing (see PCT Publication 88-10315).

Detection Methods

The method by which the synthesized and accumulated RNA transcripts are detected is dependent principally upon the desires and needs of the user. A wide variety of methods are known and will be developed in the future which can be applied to the present invention. By way of example only, a few methods will be described in detail herein. Principally, these methods are based on the production of labeled transcription product or on hybridization of the transcription product with detection of the resulting hybrids.

(1) Synthesis of labeled RNA transcripts -The addition of rNTPs, one or more of which comprise a detectable label, to the transcription mixture will result in the synthesis of RNA transcripts that also comprise the detectable label. Substances which serve as useful detectable labels are well known in the art and include radioactive isotopes, e.g., $^{32}P$, $^{3}H$, $^{125}I$, and $^{14}C$, fluorescers, chemiluminescers, chromophores, and the like, as well as ligands such as biotin and haptens which, while not directly detectable, can be readily detected by reaction with labeled forms of their specific binding partners, e.g., avidin and antibodies, respectively.

(2) Detection by hybridization with labeled probe - This approach relies upon a further hybridization step for the detection of the RNA transcripts. A variety of methods are known for the preparation of probes which comprise labels that are directly or indirectly detectable. The labels can be any of the same materials mentioned immediately above.

(3) Detection by hybridization and use of anti-hybrid reagent - The detection probe can also be selected in a manner such that the hybrids formed with the RNA transcripts are unique in the mixture and thereby selectively detectable through the use of anti-hybrid reagents as described above, e.g., antibody selective for binding DNA/RNA hybrids.

(4) Detection of RNA transcripts - The transcription products themselves can be detected in solution by separating RNA from the mixture, or by first destroying unreacted rNTPs with phosphatases, and then adding reagents that produce a detectable response to RNA, such as the bioluminescent system involving reaction with polynucleotide phosphorylase, pyruvate kinase and firefly luciferase (e.g., as described by C.P.H. Vary (1987) Nucleic Acids Res. 15:6883-6897).

Of course, since the transcription system can be designed to produce transcripts of a specified size, in such case they can be readily identified by size resolution methods such as gel electrophoresis.

The present invention will now be illustrated, but is not intended to be limited, by the following examples.

EXAMPLE 1

Demonstration of transcription of hairpin probe

Oligonucleotides having the sequence shown below were synthesized (Applied Biosystems, Foster City, CA, USA, Model 380B oligonucleotide synthesizer) for $n = 5$ and 7 using reagent supplied by the manufacturer for a phosphoramidite-type synthesis:

**TAATACGACTCACTATAG-3'**
$T_n$
**ATTATGCTGAGTGATATCCCTCCTCCTAATGGGGAGCTTAA-5'**

The oligonucleotides were purified on 20% denaturing polyacrylamide gels by electrophoresis. The purified materials were tested for their ability to produce RNA transcripts in the presence of T7 bacteriophage RNA polymerase under the conditions defined below.

Mixtures (in a 25 microliter final volume) containing 1 mM ATP, GTP, CTP, and UTP and 2 microcurie of $^{32}$P-labeled CTP (3000 curie/mM) 70 units of T7 RNA polymerase (Pharmacia, Milwaukee, WI, USA), and 1 ng of the template oligonucleotides, in 40 mM Tris-HCl (pH 8.1 at 37° C), 1 mM spermidine, 5 nm dithiothreitol, 50 g/ml bovine serum albumin, 0.01% (v/v) Triton X-100, and 80 mg/ml polyethylene glycol (8000 MW) were incubated at 37° C for 3 hours. The product was then analyzed by polyacrylamide gel electrophoresis and autoradiography.

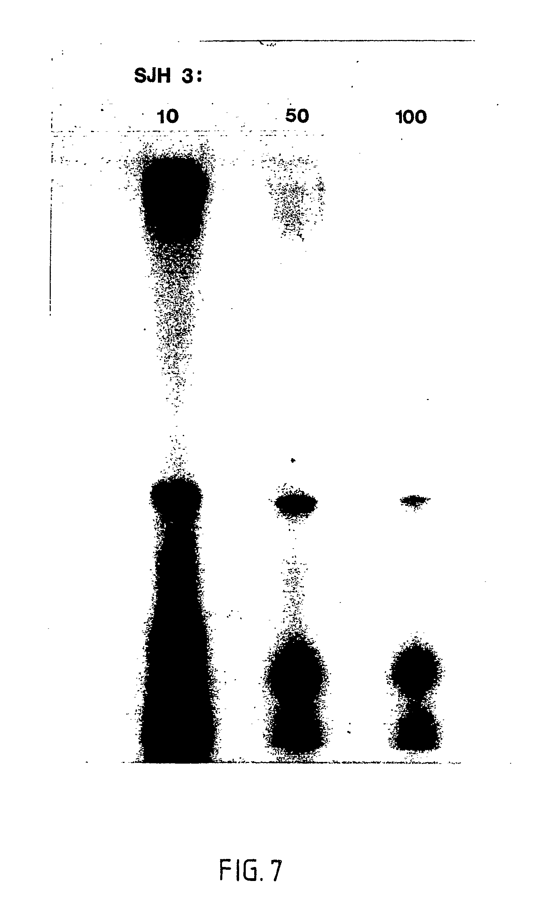

Fig. 7 shows an autoradiogram of a gel on which 10 picogram equivalents were loaded. Lanes 10, 50 and 100 indicate the amount of starting oligonucleotide in picograms used in the transcription. After transcription, all, one-fifth and one-tenth of the material was loaded for analysis. The results show production of similar amounts of RNA from similar amounts of template irrespective of initial concentration.

EXAMPLE 2

Use of a transcribable hairpin probe to detect a target nucleic acid sequence

A. Hybridization of immobilized sample nucleic acids

Transcribable hairpin oligonucleotides are prepared as in Example 1 to have the following different probe sequences added to their respective 5' ends:

5'-AGA TTT TCT AGA CAT CTT-3', and
5'-CAA GCA AGT TTA GCT CTC TCT-3'

The above probe sequences are specific to a membrane protein in the bacterium Chlamydia trachomatis. Nucleic acids from a sample to be tested for the presence of C. trachomatis are immobilized onto a solid support such as polystyrene latex. The immobilized sample nucleic acids are then hybridized with the transcribable probes under conventional conditions (Maniatis et al (1982) Molecular Cloning, Cold Spring Harbor). After washing to remove unhybridized probes, the hybridized probes are released into solution by heating at 100° C and the transcription reaction and analysis are performed as described in Example 1.

B. Hybridization of biotinylated sample nucleic acids

In this experiment, photochemically biotinylated sample nucleic acid and a control DNA were hybridized with a hairpin probe. The hybrids were captured onto streptavidin coated beads. Then the beads were treated with a DNA polymerase system to extend the 3' end of the hybridized hairpin probe. This process produces an unstable triple stranded structure from which the probe is released without heating. The released probes were then transcribed by adding the appropriate NTP mixture and the specific RNA polymerase. The transcription reaction was carried out with a mixture of $^{32}$P-labeled and unlabeled NTP and then analyzed by counting the radioactivity in the final hybrid in a scintillation counter.

A photobiotinylated 50 nanogram DNA sample (prepared by the method of Dattagupta et al, Anal. Biochem. 177:85 (1989) was denatured by heating at 100° C for 5 min., hybridized with 1 ng of hairpin probe SJH10 (having the sequence shown below):

$$\text{TAATACGACTCACTATAG-3'}$$
$$\text{T}_5$$
$$\text{ATTATGCTGAGTGATATCCCTCCTTCTACTTTAGATCTTTT-}$$
$$\text{AGAAAAAA-5'}$$

at 45° C for 30 min. in 1 M NaCl. The labeled hybrid was then captured on streptavidin agarose (pretreated with 100 μg/ml of yeast tRNA) by incubation at 37° C for 30 min.

Captured hybrid on agarose beads was washed by incubation, followed by centrifugation separation: twice with 0.1% SDS, twice with PBST (PBST: pH 7 buffer containing 35 mM $NaH_2PO_4$, 30 mM $Na_2HPO_4$ and 150 mM NaCl, 0.05% Tween 20), and once with transcription buffer (40 mM Tris, pH 8.0, 10 mM $MgCl_2$, 4 mM spermidine, 10 mM DTT, 10 mM NaCl, 50 microgram per microliter bovine serum albumin). Then a mixture of dNTPs and NTPs were added, followed by 4 units of Klenow fragment of DNA polymerase and incubated for 1 hour at 37° C. The volume of the liquid was 100 microliters. This process will release hybridized hairpin probe into solution.

Fifty (50) microliters of the solution containing the released hairpin probe is removed and 140 units of T7-RNA polymerase from Pharmacia were added, followed by 20 microcurie of alpha-$^{32}$P-labeled UTP (3000 Ci/mMole specific activity). The mixture vas incubated at 37° C for 2 hours.

Twenty-five (25) microliter of the solution containing the RNA product was then hybridized with 50 ng of a biotinylated probe oligonucleotide SJH10 at 45° C for 30 minutes. The hybrid and the unhybridized biotinylated probes were captured onto streptavidin coated magnetic particles from Dynal (Great Neck, NY, USA) by incubation at 37° C, and twice at 68° C. The beads were then counted in a scintillation counter.

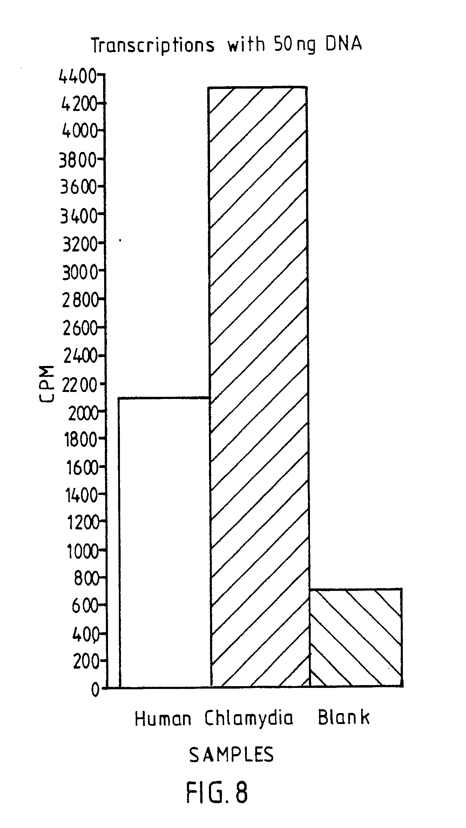

An identical protocol was followed with three sets of samples: target chlamydia DNA, control human DNA and a blank containing no nucleic acid as sample. The results are presented in Fig. 8 as a bar graph. "Human" indicates the sample was human DNA, "Chlamydia" indicates sample from chlamydia DNA, and "Blank", is for the sample with no DNA which has gone through all the procedures. The results indicate specific hybridization of the chlamydia DNA with the chlamydia specific probe.

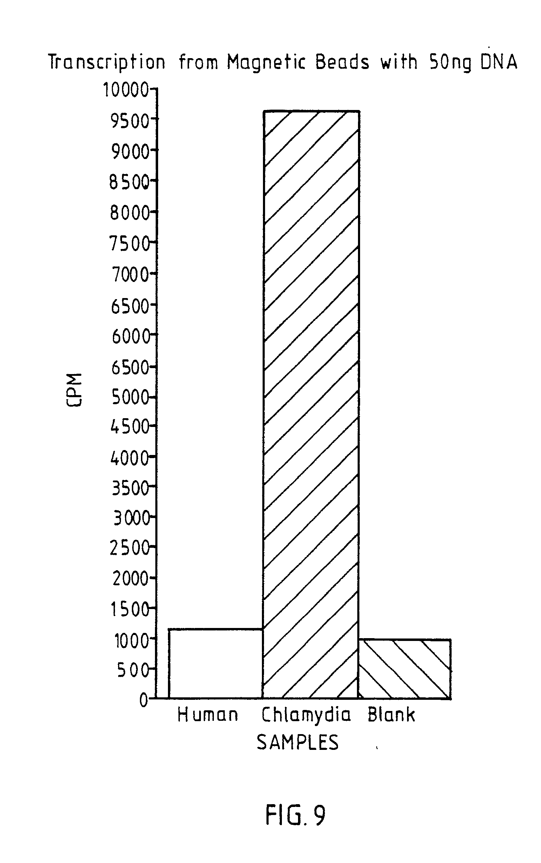

A similar method was used with magnetic beads for both stages of capture. Transcription was done without the intermediate Klenow polymerase extension treatment to release the probe into solution. The transcription reaction was performed directly with DNA on the beads. Fig. 9 shows the results with 50 ng of sample DNA.

EXAMPLE 3

Amplification of RNA targets using anti-DNA/RNA for separation

Such technique is applicable to viruses such as HIV and HRV, and also to bacteria through detection of ribosomal RNA. The following example relates to the detection of human rhinovirus (HRV 14) in a sample. A similar method can be used for the detection of other RNA analytes. The DNA probe sequence has a duplex promoter sequence and a complementary probe sequence as follows (the probe sequence is underlined and comes from Bruce et al (1989) Arch. Virol. 105:179-187):

5′-GAGGCCGGGGACTTACGATGCCCTATAGTGAGTCGTATTATTTTTTAATACGACTCACTATAG- 3′

Anti-DNA/PNA antibody is prepared according to the method of Boguslawski et al, J. Immunol. Meth. (1986) 89:123-130. The IgG fraction of the antibody is immobilized onto microspheres by incubating a mixture of antibody and a microspheres suspension at 4° C overnight according to the method of Colvin et al, Microspheres: Medical and Biological Applications, ed. Rembaum and Tokes, CRC Press (Boca Raton, FL 1988) pp. 1-13.

Suspensions containing viruses are treated with proteinase K and then extracted with phenol: chloroform:isoamyl alcohol (50:50:2) and the RNA precipitated with ethanol. The extracted RNA is then hybridized with the promoter-containing hairpin probe in a solution containing SSPE (10 mM sodium phosphate buffer, pH 7.4, 0.15 M sodium chloride, 1 mM EDTA), 5% polyethylene glycol, 0.2 mg/ml salmon sperm DNA at 40° C for 30 minutes. The antibody-immobilized microspheres are also treated with the same solution to saturate any DNA binding sites. After hybridization, the mixture is combined with the microsphere suspension and washed with the hybridization buffer and then with the transcription buffer at 37° C to remove any unhybridized probe. The microspheres with captured hybrids are then transcribed as in Example 1 and the products are analyzed by gel electrophoresis. Alternatively, the microspheres carrying hybrids can be heated to release the probes which can be transcribed and analyzed for the detection of analyte. The amount of product RNA is correlated with the amount of analyte RNA.

<div align="center">

EXAMPLE 4

</div>

<div align="center">

Amplification involving second probe and ligation in place of separation

</div>

The probe regions of the two probes used in this method (previously described in detail with reference to Fig. S) are designed such that they will hybridize with the target sequence (in this example, a chlamydial plasmid sequence) in mutually exclusive regions but with the phosphorylated 5′-end of the promoter-containing hairpin probe (see sequence below) being juxtaposed with the 3′-end of the second probe (see sequence below):

Sequence of hairpin probe (SJH P101A):

5′p-TCTCTCTCGTAATCCTCCCTATAGTGAGTCGTATTATTTTTTAATACGACTCACTATAG- 3′

Sequence of second probe (PCL5):

5′-GTCTACCACCAAGAGTTGCAAA-3′

The probes are prepared in an automated DNA synthesizer. The SJH P101 probe is kinased with polynucleotide kinase and ATP according to the method of Maniatis et al, supra, p. 122.

A sample of whole genomic chlamydial DNA containing the cryptic plasmid (Black et al (1989) Current Microbiology 19:67-74) is digested with SaCi restriction enzyme and the digest is heated to 100° C for 5 minutes for denaturation. After heating, the sample is chilled on ice. The denatured, digested DNA is then hybridized for 10 minutes with the SJH P101A and PCL5 probes at 45° C in transcription buffer as in example 1. After hybridization, 20 units of T4 ligase (Boehringer Mannheim, Indianapolis, IN, USA) and ATP (final concentration of 10 mM) are added and the ligation carried out at 37° C for 60 minutes. After ligation, the nucleic acids are optionally purified by phenol extraction and precipitated with ethanol. Transcription then proceeds in transcription buffer with 70 units of T7 RNA polymerase and the analysis as performed as in Example 1.

<div align="center">

EXAMPLE 5

</div>

<div align="center">

Further amplification of the amplified transcription products - second stage amplification

</div>

<div align="center">13</div>

The products of transcription in the above examples can be used to capture more trapscribable DNA which can be used to produce more RNA. For example, by partially substituting UTP with biotinylated UTP (Enzo, New York, NY, USA) in the examples, biotinylated RNA is produced upon transcription. This is then captured on a streptavidin solid phase. The captured RNA binds with the original promoter-containing DNA which is then further transcribed to produce more RNA under the conditions of Example 1.

The following describes a particular experiment that was performed to demonstrate a second stage RNA mediated amplification. In this method, biotinylated RNA was produced from the transcription of the hairpin probe. The biotinylated RNA was immobilized onto streptavidin coated magnetic beads. The captured RNA was then hybridized with the original hairpin probe which contained the DNA sequence complementary to the captured RNA. This produced an immobilized RNA-DNA hybrid. Such hybrid can also be obtained by first hybridizing RNA with DNA in solution and then capturing the hybrid onto the magnetic particles. The captured hairpin promoter-containing probes were then transcribed to produce RNA. The transcription reaction can be carried out by using the particles with immobilized hybrid or after denaturing the DNA into solution.

A known amount (10 femtograms) of a promoter-containing hairpin probe (designated JTH6) having the following sequence:

TAATACGACTCACTATAG-3'
T7
ATTATGCTGAGTGATATCCCTCCTTCTACTTTAGATCTTTTAGA-5'

was transcribed for three hours in a 25 microliter reaction volume containing 0.5 mM each of ATP, GTP, and CTP, 0.4 mM UTP and 0.1 mM bio-UTP (Enzo, New York, NY, USA); in a buffer containing 40 mM Tris, pH 8, 10 mM magnesium chloride, 10 mM sodium chloride, 4 mM spermidine, 1 mM DTT, 50 microgram per ml bovine serum albumin and 70 units of T7 RNA polymerase (Pharmacia, Piscataway, NJ, USA). The reaction was carried out for 3 hours at 37° C. The RNA product was then precipitated with ethanol.

The precipitated RNA was resuspended in a buffer containing 25 nM sodium phosphate, pH 7, 0.1% Tween 20, 0.1% sodium dodecyl sulfate, 40 microgram/ml herring sperm DNA. The RNA was captured onto magnetic beads in the same buffer by incubation at room temperature for 30 minutes. The beads were washed with the same buffer once and three times with the transcription buffer. The beads were then incubated at 45° C for 30 minutes in the transcription buffer containing 10 nm of SJH6 DNA for hybridization with the immobilized biotinylated RNA. After hybridization beads were washed with the same buffer at 50° C three times. The captured hybrid was then taken in the transcription buffer and transcribed at 37° C for three hours after adding 0.5 mM (final) each of ATP, CTP, GTP, UTP and 10 microcurie alpha-$^{32}$P-UTP (3000 curie/mM) and 70 units of T7 RNA polymerase.

The product RNA was then analyzed by gel electrophoresis on a denaturing 20% polyacrylamide gel. After the electrophoresis the gel was dried on a gel drier and autoradiographed on X-ray films. From the autoradiogram the position of the product could be identified and the band was cut out and counted in a scintillation counter. On one lane of the gel, a known amount of the total mixture was also loaded 15 minutes before the completion of electrophoresis. In this lane the product would not separate. The ratio between this lane and the separated product lane was taken to evaluate the reaction efficiency. Approximately 3 million-fold molar amplification was achieved.

It was also found that the efficiency of transcription could be further improved by excluding spermidine in the buffer and by adding 1 to 100 μm pGpG as the initiating nucleotide.

**Claims**

1. A method for amplifying a particular nucleic acid sequences comprising the steps of:
    (a) hybridizing said sequence of interest n a liquid mixture with a transcribable probe comprising (1) a single stranded self -complementary sequence capable of forming, under hybridizing conditions, a hairpin structure having a functional promoter region, (2) a transcribable sequence extending from the 5' end of said self-complementary sequence, which 5' sequence is transcribable in the presence of an RNA polymerase and required ribonucleoside triphosphates under said hairpin-forming hybridizing conditions,

EP 0 427 074 A2

and (3) a single stranded probe sequence substantially complementary to said sequence of interest,

(b) transcribing said probe which has become hybridized with said sequence of interest without transcribing substantially any of the unhybridized probe, such transcription taking place under hairpin-forming hybridizing conditions, and

(c) allowing the resulting transcription of the probe sequence in said hybridized probe to take place for a predetermined period of time to accumulate the resulting RNA transcription product as an amplification of said sequence of interest.

2. The method of claim 1 wherein said probe sequence is comprised in said transcribable 5′ sequence.

3. The method of claim 1 wherein said probe sequence is comprised in a sequence extending from the 3′ end of said self-complementary sequence and which is not substantially complementary to said 5′ transcribable sequence.

4. The method of any of claims 1 to 3 wherein the resulting RNA transcription product is further amplified in a second stage amplification.

5. The method of any of claims 1 to 4 wherein said polymerase is a DNA-dependent RNA polymerase, preferably T7, T3, or SP6 bacteriophage RNA polymerase, and preferably wherein said promoter region comprises the following base sequence shown in its hairpin form:

for T7,

```
 /TAATACGACTCACTATAG-3'
T
 n
 \ATTATGCTGAGTGATATC-5';
```

for T3,

```
 /ATTAACCCTCACTAAAGGGA-3'
T
 n
 \TAATTGGGAGTTAGCTTTCC-5'; or
```

for SP6,

```
 /CATACGATTTAGGTGACACTATAG-3'
T
 n
 \GTATGCTAAATCCACTGTCATATC-5';
```

wherein n is an integer from 2 to 50 inclusive.

6. A method for detecting a particular nucleic acid sequence in a test sample, comprising the steps of:

(a) hybridizing said sequence of interest in a liquid mixutre with a transcribable probe comprising (1) a single stranded self-complementary sequence capable of forming, under hybridizing conditions, a hairpin structure having a functional promoter region, (2) a transcribable sequence extending from the 5′ end of said self-complementary sequence, which 5′ sequence is transcribable in the presence of an RNA polymerase and required ribonucleoside triphosphates her said hairpin-forming hybridizing conditions, and (3) a single stranded probe sequence substantially complementary to said sequence of interest,

(b) transcribing said probe which has become hybridized with said sequence of interest without transcribing substantially any of the unhybridized probe, such transcription taking place under hairprin-forming hybridizing conditions, and

(c) allowing the resulting RNA transcription of the probe sequence in said hybridized probe to take place for a predetermined period of time to accumulate the resulting transcription product as an amplification of said sequence of interest and

(d) detecting the accumulated RNA transcription product.

7. The method of claim 6 wherein said probe sequence is comprised in said transcribable 5′ sequence.

8. The method of claim 6 wherein said probe sequence is comprised in a sequence extending from the 3′ end of said self-complementary sequence and which is not substantially complementary to said 5′ transcribable sequence.

9. The method of any of claims 6 to 8 wherein the resulting RNA transcription product is further amplified in a second stage amplification.

15

10. A reagent kit for use in amplifying a particular nucleic acid sequence, comprising:

(1) a transcribable hairpin probe comprising (1) a single stranded self-complementary sequence capable of forming, under hybridizing conditions, a hairpin structure having a functional promoter region, (2) a transcribable sequence extending from the 5´ end of said self-complementary sequence, which 5´ sequence is transcribable in the presence of an RNA polymerase and required ribonucleoside triphosphates under said hair-in-forming hybridizing conditions, and (3) a single stranded probe sequence substantially complementary to said sequence of interest, and

(2) said RNA polymerase.

FIG.1

FIG.2

FIG.3

Hairpin Probe + Sample Nucleic Acid

hybridize

(I)

separate unhybridized
probe

add polymerase and
rNTPs

Transcription Products

FIG. 4

Hairpin Probe   Second Probe + Sample

hybridize

(II)

ligate

(III)

add polymerase and
rNTPs

b'   c'        b'   c'   h'

Transcription Products

FIG.5

FIG. 6

FIG. 7

**Transcriptions with 50 ng DNA**

FIG. 8

Transcription from Magnetic Beads with 50ng DNA

FIG. 9